(19) Europäisches Patentamt
European Patent Office
Office européen des brevets

(11) Publication number: **0 233 733 A2**

(12) **EUROPEAN PATENT APPLICATION**

(21) Application number: 87301043.3

(22) Date of filing: 05.02.87

(51) Int. Cl.[4]: **C 07 D 209/48**
C 07 C 101/453, C 07 C 99/00

(30) Priority: 19.02.86 JP 34474/86

(43) Date of publication of application: 26.08.87 Bulletin 87/35

(84) Designated Contracting States: BE CH DE FR GB IT LI SE

(71) Applicant: KUREHA KAGAKU KOGYO KABUSHIKI KAISHA
9-11 Horidome-cho 1-chome Nihonbashi Chuo-ku
Tokyo (JP)

(72) Inventor: Sugita, Norifumi
402 Harashima-Biru, 2-10-13 Kasuga Bunkyo-ku
Tokyo (JP)

Niimura, Koichi
31-20 Shinsayama-Haitsu, 63 Aoyagi
Sayama-shi Saitama-ken (JP)

Fujii, Masahiko
4-10-1 Iwatominami
Komae-shi Tokyo (JP)

Furusho, Takao
1-6-13 Asahi-machi
Machida-shi Tokyo (JP)

Yoshikumi, Chikao
2-19-46 Higashi
Kunitachi-shi Tokyo (JP)

(74) Representative: Woods, Geoffrey Corlett et al
J.A. KEMP & CO. 14 South Square Gray's Inn
London WC1R 5EU (GB)

(54) **Process for producing N-phthaloyl-p-nitro-L-phenylalanine.**

(57) N-Phthaloyl-p-nitro-L-phenylalanine, an intermediate in the production of melphalan, or an ester thereof is prepared by reacting p-nitro-L-phenylalanine or an ester thereof with an N-substituted phthalimide.

EP 0 233 733 A2

## Description

"PROCESS FOR PRODUCING N-PHTHALOYL-p-NITRO-L-PHENYLALANINE"

The chemical structure of melphalan which has been known as an excellent anti-tumor agent is p-bis(2-chloroethyl)-amino-L-phenylalanine, and to exhibit the anti-tumor effect, it is necessary that this compound is in the L-form, i.e., melphalan. Its D-form is called as medphalan and its racemic-(DL)-form is called as sarcolysine and although every one of these two substances is said to be an anticancer agent, its anticancer activity is far inferior to that of melphalan.

British Patent No. I,377,336 discloses a process wherein sarcolysine (DL-form) is at first produced and melphalan (L-form) is isolated from sarcolysine. However, the process is not industrially good, because the D-form which is low in necessity is synthesized to the last step.

Also Chemical Abstract, 83-97932e discloses a process for obtaining melphalan by similarly isolating melphalan from sarcolysine.

U.S. Patent No. 3,032,585 discloses a process wherein the intermediate compound, N-acetyl-p-nitro-DL-phenylalanine, is treated by brucine, etc. to separate thereof into the D-form and the L-form and melphalan is obtained from the thus separated L-form by conventional process. In this process too, the D-form and the L-form is separated to each other during the course of synthesizing melphalan, and in the point that the D-form which is low in necessity is synthesized to a certain extent, the process is not industrially profitable.

On the other hand, a process for synthesizing melphalan has also been described in F. BERGEL (J. Chem. Soc., Part I, 2409 - 24I7, I954) and U.S. Patent No. 3,032,584. The process is not the process of separating the DL-form into the D-form and the L-form as in the above-mentioned processes. As an example of this process, the following process is shown from Example 3 of U.S. Patent No. 3,032,584:

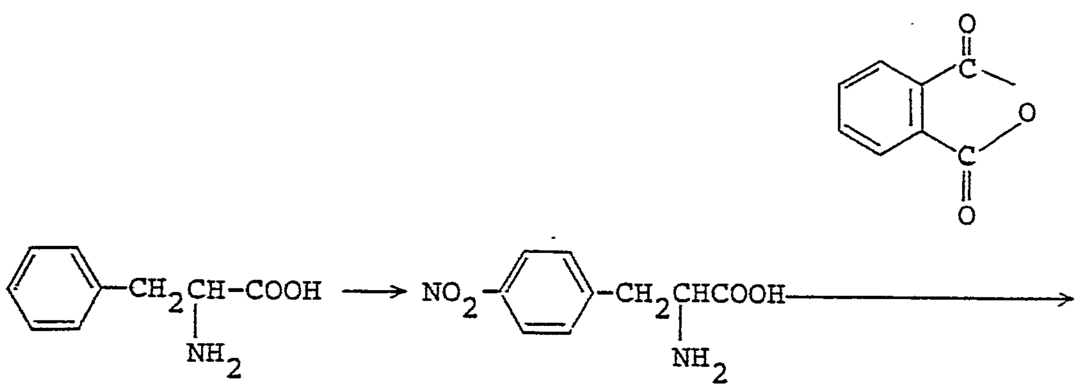

I

II

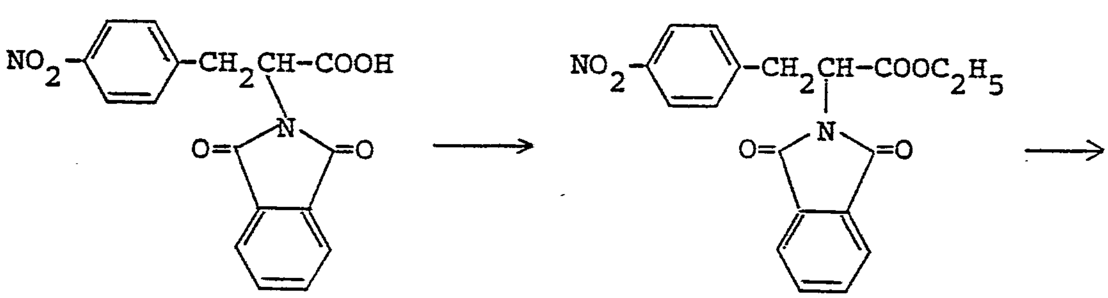

III

IV

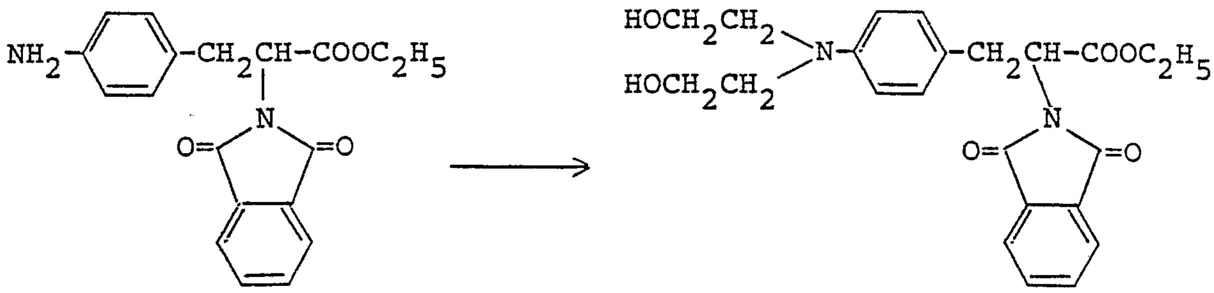

V VI

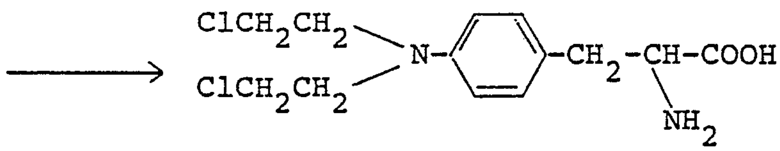

VII

The largest defect of the process lies in the loss of the optical activity of compound III by the reaction of introducing a protective group into compound II.

Namely, since in the reaction of introducing a phthaloyl group into compound II, a condition of heating and refluxing compound II and phthalic anhydride in pyridine has been adopted, the steric configuration of the alpha-position of compound III cannot be retained. Alternatively, an ethyl ester of compound II and phthalic anhydride can be reacted in benzene to obtain N-O′-carboxybenzoyl-p-nitro-L- and -D-phenylalanine ethyl esters, and in the next step, the ethyl esters are heated under a reflux condenser in an ethanolic hydrochloric acid to obtain the ethyl ester of compound III. It is also difficult to retain the steric configuration of the alpha-position by this alternative process.

According to U.S. Patent No. 3,032,584, the thus obtained compound, p-bis(2-chloroethyl)-aminophenylalanine shows the value of $[\alpha]^{25}_{D}$ $+ 7.5 \pm 0.5°$ (C: l.33, IN HCl) and the value clearly shows the occurrence of racemization in the product. Namely, the steric configuration of the alpha-position of compound III has not been retained and the optical activity of compound III has been lost.

As a result of the present inventors' earnest studies for finding a process for producing compound III from compound II in a high yield without losing the optical activity of compound III, it has been found that their object can be attained by reacting N-substituted phthalimide with compound II and based on this finding, the present invention has been completed.

The object of the present invention lies in the synthesis of N-phthaloyl-p-nitro-L-phenylalanine without losing the optical activity of p-nitro-L-phenylalanine and in a high efficiency.

Furthermore, the object of the present invention lies in the synthesis of N-phthaloyl-p-nitro-L-phenylalanine which is the intermediate raw material for producing melphalan which is an excellent anticancer agent.

Still further, the object of the present invention is to provide a process for synthesizing N-phthaloyl-p-nitro-L-phenylalanine from p-nitro-L-penylalanine while using an N-substituted phthalimide.

By nitrating L-phenylalanine shown by the formula (I) through a conventional method, for instance, nitration with nitric acid:

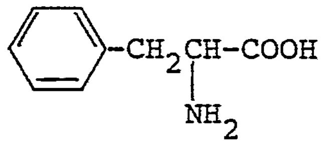

(I)

p-nitro-L-phenylalanine shown by the formula (II) is obtained:

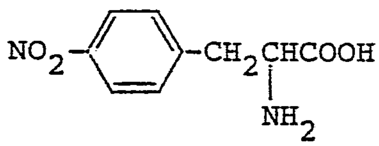

(II)

By bringing compound II (including a salt thereof) into reaction with N-(alkyloxycarbonyl)phthalimide, N-(aryloxycarbonyl)phthalimide, N-(carboxymethyl)phthalimide, a salt thereof and an ester thereof, N-(carboxyethyl)phthalimide, a salt thereof and an ester thereof, N-(alkyl)phthalimide or N-(aryl)phthalimide, the compound shown by the formula (III) is obtained:

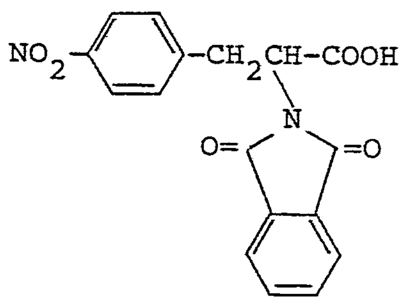

(III)

In the above-mentioned case, as a solvent for the reaction, an organic- or inorganic solvent, for instance, benzene, toluene, xylene, dioxane, methylene chloride, DMSO, DMF, DME, pyridine, water, formic acid, chloroform, acetone, THF, acetic acid, etc. may be used.

The above-mentioned reaction is carried out at a temperature of from -10 to 40°C, preferably from 0 to 20°C, and as a more preferable method, the reaction is carried out at a low temperature in the initial stage and then at a high temperature in the following stages. Namely, in the initial stage, the reaction is carried out at a temperature of from -10 to 10°C and then the reaction is continued at a temperature of from 11 to 40°C. The total reaction time period is from 5 minutes to 24 hours, preferably from 10 minutes to 4 hours.

In the above-mentioned reaction system, sodium carbonate, copper carbonate, potassium carbonate, calcium carbonate, sodium hydrogen carbonate and the like may be added.

In the next step, the compound III is esterified to obtain compound IV. The esterification may be carried out on compound II first and then bring into reaction with N-substituted phthalimide to get compound IV.

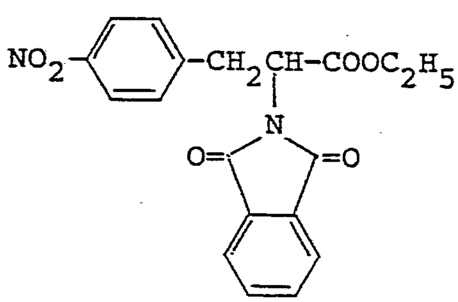

(IV)

Thereafter, compound IV is reduced in the presence of palladium-calcium carbonate ($Pd\text{-}CaCO_3$), platinum

oxide ($PtO_2$), palladium-carbon (Pd-C) or Raney nickel to obtain amino compound V. Then, compound V is brought into reaction with ethylene oxide to obtain compound VI and compound VI is chlorinated to obtain melphalan, compound VII.

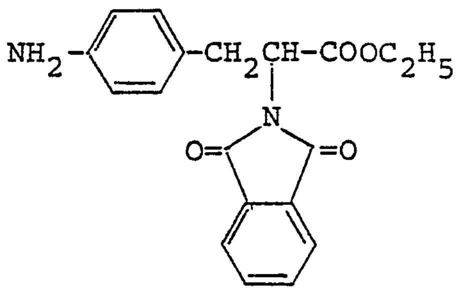

(V)

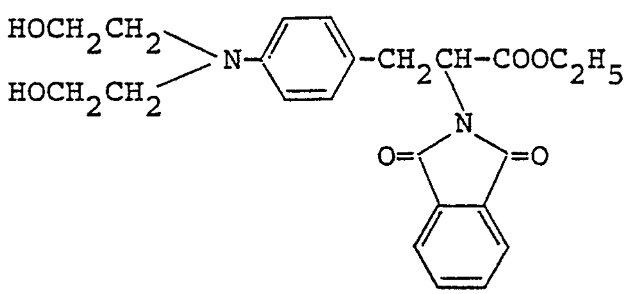

(VI)

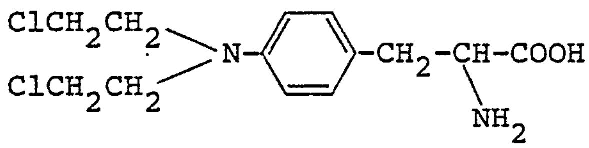

(VII)

Melphalan, p-bis (2-chloroethyl)-aminophenylalanine, produced by the above-mentioned process shows the minus value of $[\alpha]_D^{25}$ = -31.25° (C: 0.16, MeOH) as is shown later in Example 2.

On the contrary, the product obtained in Example 3 of U.S. Patent No. 3,032,584 shows the plus value of $[\alpha]_D^{25}$ = 7.5 ± 0.5. These facts show that melphalan obtained by using the process of the present invention is far rich in the L-body as compared to the product obtained by the process according to U.S. Patent No. 3,032,584, and show clearly the difference between the two processes.

In addition, it is a matter of course that in the case of using compound I of the D-form or the DL-form, the respective compound VII, namely compound VII of the D-form or that of the DL-form, are obtained.

According to the process of the present invention, N-phthaloyl-p-nitro-L-phenylalanine or its ethyl ester is obtained without losing the optical activity of p-nitro-L-phenylalanine and in a high yield.

The present invention will be explained more in detail while referring to the non-limitative Examples as follows.

EXAMPLE 1:

In a 10-litre reaction vessel, 133.1 g (1.25 mol and 1.25 equivalent) of sodium carbonate and 2.9 litres of water were introduced, and the content of the reaction vessel was stirred to dissolve sodium carbonate into water. After ice-cooling the reaction vessel to a temperature of lower than 10°C, 210 g (1 mol and 1 equivalent) of p-nitro-L-phenylalanine were introduced into the reaction vessel and the content thereof was stirred to suspend p-nitro-L-phenylalanine in the solution. Into the thus formed mixture, 314.5 g (1.43 mol and 1.43 equivalent) of N-ethoxycarbonylphthalimide were added. Thereafter, the content of the reaction vessel was stirred for 15 minutes at a temperature of lower than 10°C and the stirring was continued for a night at room temperature. After the reaction was over, the reaction product was adjusted to pH of 2.5 by about 329 ml of 6N HCl aqueous solution, and the thus formed precipitate was collected by filtration and washed with water. After compressing and drying the thus washed precipitate, it was dissolved in about 2 litres of ethanol by heating to 60°C.

After adding activated carbon to the thus formed ethanolic solution to adsorb the colored materials, the solution was filtered and the filtrate was left to cool by itself, and by leaving the solution of the crystals for a

night at 4°C, 237.3 g of N-phthaloyl-p-nitro-L-phenylalanine were obtained in a yield of 69.8%. The melting point of the thus obtained product was from 209 to 212°C.

The elementary analytical values of the thus obtained product were as follows:

| | C | H | N |
|---|---|---|---|
| Found (%) | 60.0 | 3.2 | 8.0 |
| Calcd. as $C_{17}H_{12}O_6N_2$ (%) | 60.0 | 3.5 | 8.2 |

The product showed the minus value of $[\alpha]_D^{23}$ = -230.2° (C:1.0, MeOH).

In the case where N-phenyloxycarbonylphthalimide was used in place of N-ethoxycarbonylphthalimide, a similar result was obtained.

EXAMPLE 2:

Synthesis of L-p-bis(2-chloroethyl)-aminophenylalanine was carried out while using N-phthaloyl-p-nitro-L-phenylalanine ($[\alpha]_D^{23}$ of -230.2°) obtained in Example I as a starting material as follows:

2-a) After cooling 3.1 litres of ethanol by iced water, gaseous hydrogen chloride was blown into the thus cooled ethanol for 2 hours. Thereafter, 340 g (1 mol) of N-phthaloyl-p-nitro-L-phenylalanine were suspended in the thus treated ethanol and the suspension was heated to 90°C and refluxed for 2 hours. After the reaction was over, the reaction mixture was condensed under a reduced pressure with an evaporator, and 550 ml of benzene were added to the condensate. Hydrogen chloride was removed from the mixture by azeotropic distillation with benzene. The white crystals obtained by drying and solidifying the thus treated product was dissolved into 1.81 litres of n-propanol and charcoal was added to the thus formed solution. After filtrating the solution, 600 ml of n-hexane were slowly added to the filtrate for carrying out crystallization and after leaving the mixture for a night at 4°C, the mixture was subjected to filtration to obtain 293.6 g of the ethyl ester of N-phthaloyl-p-nitro-L-phenylalanine of $[\alpha]_D^{23}$ of -212° in a yield of 79.8%.

2-b) After dissolving 184 g (0.5 mol) of the ethyl ester of N-phthaloyl-p-nitro-L-phenylalanine into 640 ml of ethyl acetate, the thus prepared solution was introduced into an autoclave.

After adding 640 ml of methanol and 4.6 g of platinum dioxide into the autoclave, the ethyl ester was subjected to reduction for about 3 hours under 2.5 atmospheric pressure while stirring at 400 r.p.m. After the reaction was over, the reaction mixture was filtered for removing the catalyst, and the filtrate was condensed with an evaporator. Thereafter, methanol was added to the condensate and the mixture was again subjected to condensation and drying to solid. The thus solidified material was dissolved into about 2 litres of hydrochloric acid of 1.5N, and after washing the thus formed solution two times with each 632 ml of diethyl ether to remove the unreacted subtances, the etheric layer and the hydrochloric acid solution layer were separated. After adjusting the pH of the hydrochloric acid solution to 8 by aqueous ammoniacal solution under ice-cooling, the thus treated solution was subjected to extraction by 1.5 litres of chloroform. After washing the extract with water and drying the washed extract, the thus dried extract was condensed under a reduced pressure. After dissolving the condensate in 402 ml of methanol, the solution was treated with activated carbon and 131 ml of water was added to the solution. 126 g of the ethyl ester of N-phthaloyl-p-amino-L-phenylalanine of $[\alpha]_D^{23}$ of -153.1° in a yield of 74.6% was obtained.

2-c) Into 1.34 litres of acetic acid, 169 g (0.5 mol) of the ethyl ester of N-phthaloyl-p-amino-L-phenylalanine were dissolved, and 1.34 litres of water were added to the solution. Into the thus prepared mixture, 395 ml of ethylene oxide were slowly added and the resultant mixture was stirred for a night at room temperature. After the reaction was over, 3.2 litres of water and 320 ml of ethyl acetate were added to the reaction mixture. After adjusting the pH of the resultant mixture to 7 by adding sodium hydrogen carbonate, 1.34 litres of ethyl acetate was added to the mixture to subject thereof to extraction. After washing the extract with water, the washed extract was dried and condensed under a reduced pressure to obtain 106.5 g of the crude ethyl ester of N-phthaloyl-p-bis(2-hydroxyethyl)-amino-L-phenylalanine.

2-d) Into 1.52 litres of benzene, 106.5 g of the crude ethyl ester of N-phthaloyl-p-bis(2-hydroxyethyl)-amino-L-phenylalanine obtained in 2-c) were dissolved, and after adding 351 ml of phosphorus oxychloride into the solution, the thus formed mixture was heated for one hour at 100°C under a reflux condenser. After the reaction was over, the reaction mixture was condensed and dried to a solid state. After dissolving the thus dried solid product into 1.22 litres of conc. hydrochloric acid, the solution was heated for 7 hours at 100°C under a reflux condenser and left to stand for a night. After removing the thus deposited crystals by filtration, the filtrate was condensed to 1/3 in volume. After introducing the condensate into 1.67 litres of water, the pH of the mixture was adjusted to 8 by an aqueous ammoniacal solution under cooling and the thus precipitated crystals were collected by filtration.

While using methanol in a ratio of 100 ml of methanol to 1 g of the thus obtained crude crystals, the crude crystals were dissolved in methanol at 60°C. After treating the thus formed solution with charcoal, the solution was filtered and the filtrate was subjected to condensation until the crystals appeared. After

stopping the condensation, the solution was left to stand for a night at 4° C to obtain 23.65 g of the purified crystals of L-p-bis(2-chloroethyl)-amino-phenylalanine (melphalan) in a yield of 31.6%. The thus obtained compound melts at a temperature of from 172 to 174° C and showed $[\alpha]_D^{25}$ of -31.25° (C: 0.16, MeOH), showing that this compound is melphalan.

## Claims

1. A process for producing N-phthaloyl-p-nitro-L-phenylalanine or an ester thereof, characterised in that an N-substituted phthalimide is reacted with p-nitro-L-phenylalanine or an ester thereof.
2. A process according to claim 1, wherein the reaction is carried out at a temperature from -10 to 40° C.
3. A process according to claim 2, wherein the reaction is at first carried out at a temperature of from -10 to 10° C and then carried out at a temperature from 11 to 40° C.
4. A process according to any one of the preceding claims, wherein the said ester is an alkyl ester.
5. A process according to claim 4, wherein the said alkyl ester is the ethyl ester.
6. A process according to any one of the preceding claims, wherein the said N-substituted phthalmide is an N-(alkyloxycarbonyl)phthalimide.
7. A process according to claim 6, wherein the said N-(alkyloxycarbonyl)phthalmide is N-(ethoxycarbonyl)phthalimide.
8. A process according to any one of the preceding claims, wherein the said N-substituted phthalimide is an N-(aryloxycarbonyl)-phthalimide.
9. A process according to claim 8, wherein the said N-(aryloxycarbonyl)phthalimide is N-(phenyloxycarbonyl)phthalimide.
10. A process for producing melphalan by converting N-phthaloyl-p-nitro-L-phenylalanine or an ester thereof into melphalan, characterised by preparing the N-phthaloyl-p-nitro-L-phenylalanine or ester thereof by a process as claimed in any one of the preceding claims.